# EUROPEAN PATENT APPLICATION

(11) **EP 4 275 584 A1**
(43) Date of publication of application: **15.11.2023**
(21) Application number: 21917319.2
(22) Date of filing: 29.12.2021
(51) Int. Cl.: A61B 1/005

(54) **BENDING TUBE FOR ENDOSCOPE**

(30) Priority: 07.01.2021 CN 202120034208 U
(71) Applicant: Chen, Yongrui, Shanghai 201108 (CN)
(72) Inventor: KUANG, Jin, Shanghai 201108 (CN); LI, Yongqiang, Shanghai 201108 (CN)
(74) Representative: Danubia Patent & Law Office LLC
(86) International application number: PCT/CN2021/142304
(87) International publication number: WO 2022/148280

(57) **Abstract**

The invention relates to an endoscopic bending tube, which comprises a bending tube, and a tubular steel wire rope welding section and a tubular connect collar fixing section respectively connected to both ends of the bending tube, the bending tube includes several tubular unit segments connected in series; Two adjacent unit segments are connected by an embedded structure , the embedded structure includes a first rotation part and a second rotation part respectively disposed at two ends of the unit segment; Both sides of the inner wall of the unit segments are provided with through holes at corresponding positions for the insertions of the steel wire ropes. The invention has the advantages of simple structure, low cost, high reliability and good bending shape.

## Description

### BACKGROUND OF THE INVENTION

### 1. Technical Field

The invention relates to the technical field of endoscopic instruments, in particular to an endoscopic bending tube.

### 2. Description of the Related Art

Endoscope has been widely used in the medical field or the industrial field, which can be used in a minimally invasive inspection of patients and industrial products. Since the camera of the endoscope for performing the examination is often disposed at the front end of the outer tube, if the camera is rotated or the shooting field of view is changed, a controllable bending tube must be disposed at the insertion end of the outer tube, so that the shooting field of view of the camera is changed along with the bending of the insertion end of the outer tube.

The endoscopic bending tube in the prior art all rivets each bending unit segment to achieve bending, but the riveting structure is complicated. The riveting structure has many parts, is easily damaged, and a poor bending form, so that an endoscopic bending tube with simple structure, low cost, high reliability, and good bending form is required.

### SUMMARY OF THE INVENTION

The invention relates to an endoscopic bending tube, which comprises a bending tube, and a tubular steel wire rope welding section and a tubular connect collar fixing section respectively connected to both ends of the bending tube, the bending tube includes several tubular unit segments connected in series; Two adjacent unit segments are connected by an embedded structure , the embedded structure includes a first rotation part and a second rotation part respectively disposed at two ends of the unit segment; Both sides of the inner wall of the unit segments are provided with through holes at corresponding positions for insertions of steel wire ropes.

In some embodiments, the first rotating part includes a first ring provided with an opening and an annular first groove arranged at the outer side of the first ring.

In some embodiments, the second rotating part includes a second ring provided with an opening and an annular second groove arranged at the inner side of the second ring; The second ring can be inserted into the first groove; The first ring can be inserted into the second groove.

In some embodiments, the upper and the lower parts of the two ends of the unit segment are bevels.

In some embodiments, at the front part of the endoscopic bending tube, the angle formed with the upper parts and the angle formed with the lower parts of the adjacent unit segments both have A degree;
At the middle part of the endoscopic bending tube, the angle formed with the upper parts of the adjacent unit segments has B degree, and the angle formed with the lower parts of the adjacent unit segments has C degree;
At the back part of the endoscopic bending tube, the angle formed with the lower parts of the adjacent unit segments has D degree; The size sequence of the angle is C>B>A>D.

In some embodiments, two riveting positions are arranged respectively at the opposite sides of the circumference of the steel wire rope welding section, and a processing hole is arranged adjacent to one of the riveting positions.

In some embodiments, two processing holes are arranged respectively at the opposite sides of the circumference of the connect collar fixing section.

In some embodiments, the bending tube is a stainless steel tube cut by laser.

The invention has the advantages of convenient processing, low manufacturing cost, good bending shape, high bending flexibility and reliable quality.

### BRIEF DESCRIPTION OF THE DRAWINGS

The present invention will become more fully understood from the detailed description given herein and the accompanying drawings which are given by way of illustration only, and thus are not limitative of the present invention, and wherein:
FIG. 1 is a structural schematic diagram of an embodiment of the invention;
FIG. 2 is a structural schematic diagram of the unit segment of the invention;
FIG. 3 is a schematic view of the use state of an embodiment of the invention being bent to the small angle side;
FIG. 4 is a schematic view of the use state of an embodiment of the invention being bent to the large angle side.

### DETAILED DESCRIPTION OF EMBODIMENTS OF THE INVENTION

For the purpose of more clearly understanding the technical contents of the invention, the following embodiment examples are cited for detailed explanation.

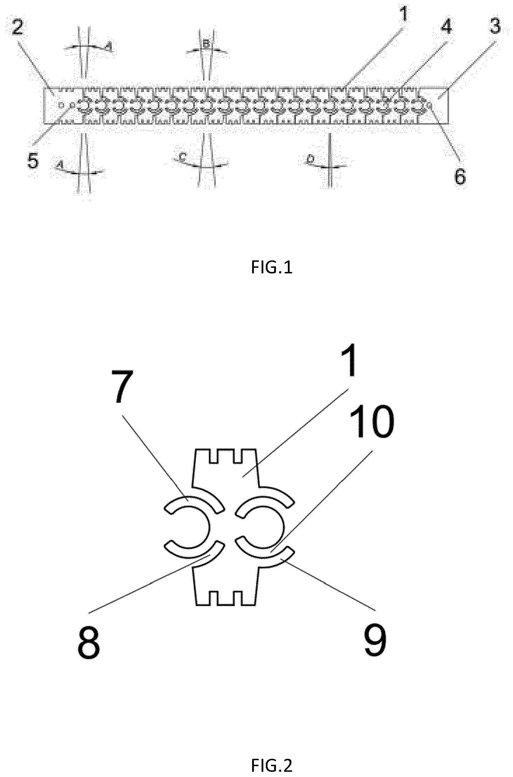

FIG. 1 is a structural schematic view of a preferred embodiment of the endoscopic bending tube of the invention. In this embodiment, the endoscopic bending tube comprises a bending tube, and a tubular steel wire rope welding section 2 and a tubular connect collar fixing section 3 respectively connected to both ends of the bending tube, wherein the bending tube includes 19 tubular unit segments connected in series. Two adjacent unit segments 1 are connected by an embedded structure 4, wherein the embedded structure 4 includes a first rotation part and a second rotation part respectively disposed at two ends of the unit segment, and the adj acent unit segments 1 are connected through the embedment of the first rotation part and the second rotation part to achieve the relative rotation of the adjacent unit segments 1. The upper and the lower parts of the two ends of the unit segment 1 are bevels. At the front part of the endoscopic bending tube, the angle formed with the upper parts and the angle formed with the lower parts of the adjacent unit segments 1 both have A degree. In a preferred embodiment, the front part of the endoscopic bending tube refers to the steel wire rope welding section and the unit segment connected thereto.

At the middle part of the endoscopic bending tube, the angle formed with the upper parts of the adj acent unit segments 1 has B degree, and the angle formed with the lower parts of the adjacent unit segments 1 has C degree. At the back part of the endoscopic bending tube, the angle formed with the upper parts of the adjacent unit segments has B degree, and the angle formed with the lower parts of the adjacent unit segments has D degree; The size sequence of the angles is C>B>A>D. That is to say, the side on which the angle of C degree is located is the large angle side, and the side on which the angle of B degree is located is the small angle side.

In a preferred embodiment, two riveting positions 5 are arranged respectively at the opposite sides of the circumference of the steel wire rope welding section, and a processing hole 6 is arranged adjacent to one of the riveting positions 5. In the endoscopic bending tube, two processing holes 6 are arranged respectively at the opposite sides of the circumference of the connect collar fixing section. Both sides of the inner wall of the unit segments are provided with through holes at corresponding positions for insertions of steel wire ropes.

In the embodiment of the unit segment as shown in FIG. 2, the first rotating part includes a first ring 7 provided with an opening and an annular first groove 8 arranged at the outer side of the first ring 7. The second rotating part includes a second ring 9 provided with an opening and an annular second groove 10 arranged at the inner side of the second ring 9. The second ring 9 can be inserted into the first groove 8, and the second ring 9 can rotate in the first groove 8. The first ring 7 can be inserted into the second groove 10, and the first ring 7 can rotate in the second groove 10.

In a preferred embodiment, the endoscopic bending tube is a stainless steel tube cut by laser, and the bending is realized by cutting perpendicular to the length of the bending tube towards the axis of the metal tube with laser to form the rings and the grooves.

In a preferred embodiment, the end of the steel wire rope welding section 2 is equipped with the head base of the endoscope. The head base of the endoscope is generally equipped with working parts such as CMOS image sensor, light guide hole, working channel, water jet hole, etc.

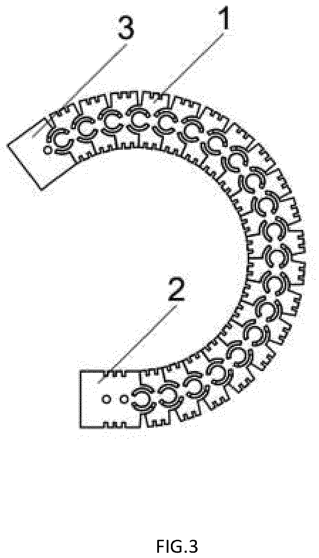

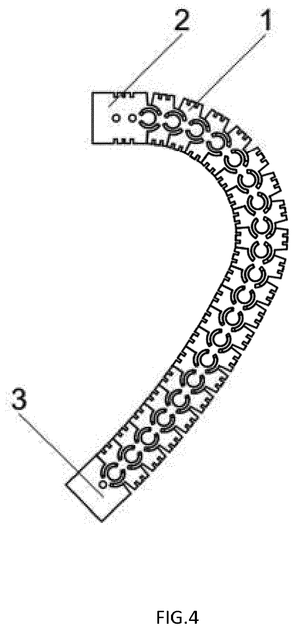

In a preferred embodiment, two steel ropes are arranged inside the endoscopic bending tube, and the two steel ropes pass through the through holes arranged on the inner wall of the unit segment 1 and are fixed on the riveting positions of the steel wire rope welding section 2. The through hole is a hollow pipe which is arranged along the length direction of the bending tube, and the axis of the through hole is in the same plane as the rotation direction of the bending tube. When the wire rope at the small angle side is pulled, the endoscopic bending tube will bend at the small angle side, as shown in FIG. 3, which shows the uniform curvature radius under the maximum bending state of the endoscopic bending tube, in this situation, the angles formed with the upper parts of the adjacent unit segments all have B degree. When the wire rope at the large angle side is pulled, the endoscopic bending tube will bend at the large angle side, as shown in FIG. 4, which is the bending state at the large angle side of the endoscopic bending tube. As shown in FIG. 3 and FIG. 4 which are the schematic views of the use states of the embodiment, the endoscopic bending pipes in the bending states both have smooth arc shapes.

In the prior art, the unit segments of the endoscopic bending tube are connected by rivet structures, the endoscopic bending tube with this structure cannot automatically spring back to the original state after being rotated. The endoscopic bending tube of the invention is integrally cut from a stainless steel tube, rather than being connected by individual unit segments after the stainless steel tube is cut into the individual unit segments. The endoscopic bending tube is an integral component, when the wire rope is pulled to bend the endoscopic bending tube, a certain resilience will be generated. When the wire rope is released, the endoscopic bending tube will automatically return to the original state. The invention improves the convenience and the feeling in use.

The endoscopic bending tube of the invention is cut by a stainless steel tube, which has the advantages of convenient processing, low manufacturing cost, good bending shape, high bending flexibility and reliable quality.

In this description, the invention has been described with reference to specific embodiments thereof. However, it is evident that various modifications and variations may be made without departing from the spirit and scope of the invention. Accordingly, the description and the drawings are to be considered as illustrative rather than restrictive.

### REFERENCE SIGNS LIST

- 1: unit segment
- 2: steel wire rope welding section
- 3: connect collar fixing section
- 4: embedded structure
- 5: riveting position
- 6: processing hole;
- 7: first ring
- 8: first groove
- 9: second ring
- 10: second groove

## Claims

1. An endoscopic bending tube, comprising a bending tube, and a tubular steel wire rope welding section and a tubular connect collar fixing section respectively connected to both ends of the bending tube, wherein
the bending tube includes several tubular unit segments connected in series;
two adjacent unit segments are connected by an embedded structure, the embedded structure includes a first rotation part and a second rotation part respectively disposed at two ends of the unit segment;
both sides of the inner wall of the unit segments are provided with through holes at corresponding positions for insertions of steel wire ropes.

2. The endoscopic bending tube according to claim 1, wherein the first rotating part includes a first ring provided with an opening and an annular first groove arranged at the outer side of the first ring.

3. The endoscopic bending tube according to claim 2, wherein the second rotating part includes a second ring provided with an opening and an annular second groove arranged at the inner side of the second ring; The second ring can be inserted into the first groove; The first ring can be inserted into the second groove.

4. The endoscopic bending tube according to claim 1, wherein the upper and the lower parts of the two ends of the unit segment are bevels.

5. The endoscopic bending tube according to claim 4, wherein at the front part of the endoscopic bending tube, the angle formed with the upper parts and the angle formed with the lower parts of the adjacent unit segments both have A degree;
at the middle part of the endoscopic bending tube, the angle formed with the upper parts of the adjacent unit segments has B degree, and the angle formed with the lower parts of the adjacent unit segments has C degree;
at the back part of the endoscopic bending tube, the angle formed with the upper parts of the adj acent unit segments has B degree, and the angle formed with the lower parts of the adj acent unit segments has D degree; the size sequence of the angles is C>B>A>D.

6. The endoscopic bending tube according to claim 1, wherein two riveting positions are arranged respectively at the opposite sides of the circumference of the steel wire rope welding section, and a processing hole is arranged adjacent to one of the riveting positions.

7. The endoscopic bending tube according to claim 1, wherein two processing holes are arranged respectively at the opposite sides of the circumference of the connect collar fixing section.

8. The endoscopic bending tube according to claim 1, wherein the bending tube is a stainless steel tube cut by laser.
